# EUROPEAN PATENT APPLICATION

(11) **EP 1 726 320 A1**
(43) Date of publication of application: **29.11.2006**
(21) Application number: 05104518.5
(22) Date of filing: 26.05.2005
(51) Int. Cl.: A61L 27/30, A61L 27/32, A61L 27/50

(54) **Coated implant system with chemically bonded ceramic material**

(71) Applicant: Doxa AB, 754 51 Uppsala (SE)
(72) Inventor: HERMANSSON, Leif, 260 42, Mölle (SE)
(74) Representative: Holmberg, Nils Anders Patrik

(57) **Abstract**

The present invention generally relates to dental and orthopaedic implants, and more specifically to an implant coated with a chemically bonded ceramic, having a modified substrate surface to improve adhesion of the implant coating to the implant material, wherein the coating includes a gradient zone also containing the material of the implant device wherein the concentration of the implant device material decreases in a direction towards the surface of the coating, and a method of preparing the coated implant as a method of implanting the implant device.

## Description

### TECHNICAL FIELD

The present invention generally relates to dental and orthopaedic implants, and more specifically to an implant coated with a chemically bonded ceramic, having a modified substrate surface to improve adhesion of the implant coating to the implant material, wherein the coating includes a gradient zone also containing the material of the implant device wherein the concentration of the implant device material decreases in a direction towards the surface of the coating.

### BACKGROUND ART

For implants that are to interact with the human body, implant materials that due to their biocompatibility provide an optimal fixation or anchoring of the implant to the biological tissue, e.g. bone, are advantageous. To allow for early loading of an implant and to reduce the risk for long term loosening, high quality fixation is important. In the case of a coated implant, the anchoring of the coating to the implant surface is often the weak spot of the implant system. The adhesion strength of the coating to the implant is often too low, and stress is often found in the contact zone due to mismatch in properties of the implant material, on the one hand, and the coating material, on the other hand.

The contact zone of an implant to the living tissue is critical and often controls the survival (the life time) of an implant. The degree of bone ingrowth towards the implant, the rate of ingrowth, and possible integration of tissue with implant control the behaviour of the contact zone. By coating especially metallic implant surfaces with a layer of chemically bonded ceramics, the growth and the chemical integration of bone and implant can be improved. Such examples include plasma-sprayed hydroxyapatite (10-100 µm thick layers) and physical vapour deposited (PVD) hydroxyapatite (0.1-10 µm thick layer). A problem which has reduced the use of these coatings is the low adhesion of the coating to the implant.

Accordingly, it is an object of the present invention to provide a coated implant exhibiting improved adhesion of the coating to the surface of the implant material. Also, the implant system should fulfil the requirements on implantation system and materials, such as desired porosity and desired thickness to optimize the mechanical property profile, i.e. high shear strength of the layer towards the substrate and reduced thickness of the layer to eliminate larger defects in the layer/coating.

For an implant material having a coating of a chemically bonded ceramic material this object has been achieved by means of including a gradient zone in the coating, which zone also contains the implant material and in which zone the concentration of the implant material decreases in a direction towards the surface thereof as defined in claim 1.

### SUMMARY OF THE INVENTION

Accordingly, in a first aspect the invention relates to an implant having a coating of a chemically bonded ceramic material exhibiting improved adhesion to the underlying implant surface, wherein the coating comprises a gradient zone of about 1 µm or less also containing the implant material, in which zone the concentration of the implant device material decreases in a direction towards the surface of the coating.

According to the invention the bond strength (adhesion) can be increased by more than 1.3 times before hydration *in vivo* as compared to a similar material without such gradient, and by more than 40 % after hydration.

In another aspect the invention relates to a method of preparing an implant material of the present invention in which a gradient zone of a chemically bonded material is applied in a thickness of 1 µm or less, in which zone the concentration of the implant material decreases in the direction towards the surface of the coating. The gradient zone of the coating can be formed by means of conventional methods of deposition of the implant and coating materials, or by applying discrete layers of the coating material having a concentration of the implant device material gradually decreasing with each consecutive layer throughout the gradient zone. In a preferred embodiment he gradient is achieved by means of deposition, using e.g. physical vapour deposition (PVD) or chemical vapour deposition (CVD).

In a further aspect the invention relates to a method of implanting the implant of the invention into a mammal in need thereof, e.g. for veterinary use.

Further advantages and embodiments will be evident from the detailed description and appended claims.

### BRIEF DESCRIPTION OF THE DRAWING

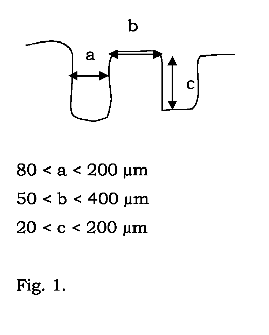

Figure 1 shows the surface structure of a surface of an implant device to be coated according to one embodiment of the invention.

### DETAILED DESCRIPTION OF THE INVENTION

The present invention relates to the anchoring of implant systems with binding agent systems of the hydrating cement type as coating material, in particular of aluminates, silicates, phosphates, fluorides, carbonates, sulphates and combinations thereof, preferably having calcium as the major cation. This in combination with a gradient zone according to the present invention yields implant fixation systems of high quality. The invention is especially intended for dental and orthopaedic implants. The implant may be metallic, polymeric or ceramic.

Examples of chemically bonded ceramics suitable for use in the present invention are those described in SE 463 493, SE 502 987, SE 514 686, SE 516 264, SE 524 494, SE 521 938, SE 524 334, SE 521 973, SE 522 749, PCT/SE 2003/001679 and PCT/SE 2004/001745, the contents of which are incorporated herein by reference.

As mentioned above, one problem which has reduced the use of these coatings is the low adhesion of the coating to the implant. The present inventors believe the low adhesion to be due to the different chemical natures of the implant and the coating material, respectively, as well as mechanical stress developed during processing due to mismatch in physical properties, e.g. in the thermal expansion coefficient. Low shear strength in the contact zone between coating and underlying implant surface has increased the risk of a fall off of the coating *in vivo* after some time, and consequently a loss of the benefit of the coating, and a possible concomitant inflammation.

The present invention aims at providing a system based on chemically bonded ceramic materials (CBC-materials), for *in vivo* anchoring of an implant to a biological tissue, where the adhesion aspects of the coating to the bulk implant material is of prime interest.

To overcome the above-mentioned adhesion problem of coatings the present invention uses an interface which is composed of both the implant material and the actual coating. Accordingly, the inventive coating includes a gradient zone wherein the concentration of the implant device material decreases in a direction towards the surface of the coating. The contact zone is thus integrated in the implant system via a gradient of both the implant material and the coating material. The gradient has a maximal thickness of one micrometer. The most optimized thickness is approximately 500 nm. The thickness should exceed 50 nm. Surprisingly the gradient increases the bond strength (adhesion) of the coating with > 1.3 times compared to that without a gradient before hydration *in vivo.* In an *in vivo* situation (i.e. after hydration in contact with living tissue) the corresponding increase exceeded 40 %, as will be demonstrated in the Examples.

Within the gradient zone an essentially continuous change in concentrations of the implant and coating materials is preferred, although a step-wise change also is conceivable. The gradient zone preferably starts from a concentration of about 100 % of the implant material extending to a concentration of no higher than 40 %, and more preferably to 0% of the implant material (i.e. 100 % coating material). In the step-wise mode the chemical composition change in every step should not exceed 20 % of the implant material.

The implant surface can be coated partially or completely with the gradient-exhibiting coating according to the invention.

The implant material used is preferably based on Ti, Ta, Co-Cr or stainless steel.

Examples of preferred chemically bonded ceramic materials are Ca-aluminates and Ca-silicates as well as phosphates and sulphates.

In another aspect the invention relates to a method of preparing the implant material of the present invention. In its most generic embodiment the inventive method includes the following steps:
a) providing an implant device;
b) applying a coating of a chemically bonded ceramic material to a surface of the implant device, wherein the coating also contains the material of the implant.

While the gradient zone also could be formed by applying discrete layers having decreasing concentration of the material of the implant, such as by means of sol-gel and similar methods for thin layer application, methods not resulting in discrete layers are preferred. Examples of preferred methods includes conventional methods of physical or chemical deposition, such as PVD, plasma spraying, CVD, and electrophoretic deposition. Such methods offer a better control of the composition of the resulting coating, and also, a more continuous gradient can be obtained. Especially preferred methods are PVD and CVD.

In a subsequent step c), preferably immediately before implantation, partial or complete hydration of the reactive coating material can be carried out.

Thereafter, in a step d), the implantation takes place. The CBC-material will then be anchored to both the implant material and the biological tissue by dissolution-precipitation and volume increase and by bioactive bonding to the surrounding tissue.

In one embodiment of the implant of the invention a surface thereof is provided with a surface structure enhancing the anchoring of the CBC coating thereto and/or promoting ingrowth of tissue into cavities in the structure. Unhydrated or partially hydrated chemically bonded ceramics of the above mentioned types can be used to fill out the cavities in the implant surface. The cavities are typically 100 µm in depth (c), 100 µm in width (a) and the distance (b) between the cavities is approximately 100 µm, but could vary within the intervals 80 <a<200, 50<b<400 and 20<c<200 µm, as shown in Figure 1. Methods for preparing this topography include laser and ultrasonic drilling.

Accordingly, in one embodiment of the inventive method the method includes the following step preceding the application of the gradient coating in step b): forming on a surface of the implant device a surface containing cavities having a width "a", a depth "c", and a distance between neighbouring cavities "b", wherein 80 < a < 200; 50 < b < 400; and 20 < c < 200 µm.

After step b) the cavities can be filled with the CBC material. The filling of the cavities can be done in several ways including slip casting, dry pressing (conventional or cold isostatic pressing) or by a paste smeared/pressed into the cavities, and left in the non-hydrated or hydrated state.

The use of a resorbable or slowly resorbable CBC-system for filling the cavities is preferred. The surface area between the cavities is preferably coated with the gradient material.

The CBC-system for the gradient material of the invention is preferably stable or slowly resorbable, and preferred systems are thus aluminates and silicates.

The final hydration occurs *in vivo* in contact with body liquid. This reaction involves a mass increase meaning that also a part of the distance between the cavities will be covered by the chemically bonded ceramic. The system with filled cavities of the mentioned size increase the adhesion strength to bone initially - within a few hours. In the case with slowly resorbable material such as chemically bonded ceramics of silicate and sulphate type the filled cavities will after some time be replaced with new formed bone tissue if the cavity size exceeds approximately 100 µm.

The present invention will now be illustrated by means examples which are provided for the purpose of illustration only and should not be construed as limiting the invention except as defined by the claims.

### EXAMPLES

### Example 1

The adhesion strength of coatings with and without gradient technology was tested according to the following;

| | | |
|---|---|---|
| Materials: | A Implant of cp Ti | Layer thickness 1 µm of Ca―Aluminate (CA) (no gradient) |
| | B Implant of cp Ti | Gradient thickness 0.5 µm and total coating thickness 1 µm (Ti 0-0.5 µm from 100 to 0 % and CA 0-0.5 µm from 0 to 100 % + an additional 0.5 µm of 100%) |
| | C Implant of cp Ti | No layer and surface treatment |

Method: The implant surface was sputtered from a Ca-aluminate-target and a Ti-target in a pulsed dc magnetron PVD equipment.

Evaluation: The tensile strength was measured according to a modified ASTM method No. F 1147-99.

Result: The shear strength increased from 26 MPa for A to > 34.5 MPa for B corresponding to an increase > 30 %.

### Example 2

The implant material used was cp Ti in the form of cylinders (length, 1 =5 mm and diameter, φ = 4 mm) and treated according to A and B procedure in Example 1 above. The implants as un-hydrated were implanted into the femur of New Zeeland white rabbits (5 kg) for 1 week and 6 weeks.

The shear strength was measured with push-out testing in a universal testing machine (Zwick). The results are shown in Table 1 below.

**Table 1. Shear strength development for differently treated cp Ti samples.**

| Material | Shear strength in MPa Time - 1 week | Shear strength in MPa Time - 6 weeks |
|---|---|---|
| A | 5 | 12 |
| B | 8 | 13 |
| C | 2 | 6 |

## Claims

1. Implant device having a surface coated with a chemically bonded ceramic material exhibiting improved adhesion of the coating to surface, **characterized in that** the coating comprises a gradient zone of about 1 µm or less also containing the implant material in which zone the concentration of the implant device material decreases in a direction towards the surface of the coating.

2. The implant device of claim 1, wherein the thickness of the gradient zone is from about 1 µm to about 50 nm, more preferably 1 µm to about 500 nm, and most preferably about 500 nm.

3. The implant device of claim 1 or 2, wherein the concentration of the implant material in the gradient zone decreases to 0%.

4. The implant device of any of the previous claims, wherein the implant material is based on Ti, Ta or Co-Cr or stainless steel.

5. The implant device of any of the previous claims, wherein the chemically bonded ceramic is selected from aluminates, silicates, phosphates, fluorides, carbonates and/or sulphates.

6. The implant device of any of the previous claims, wherein the chemically bonded ceramic is selected from Ca-aluminates, Ca-silicates, Ca-phosphates, Ca-fluoride, Ca-carbonate and/or Ca-sulphates.

7. The implant device of any of the previous claims, wherein a surface of the implant device exhibits a surface structure containing cavities having a width "a", a depth "c", and a distance between neighbouring cavities "b", wherein 80 < a < 200; 50 < b < 400; and 20 < c < 200 µm.

8. Method of producing an implant of device of claim 1, including the following steps:
providing an implant device;
applying a coating of a chemically bonded ceramic material to a surface of the implant device **characterized in that** the coating also contains the material of the implant device.

9. Method of claim 8, including the following step which precedes the application step: forming on a surface of the implant device a surface containing cavities having a width "a", a depth "c", and a distance between neighbouring cavities "b", wherein 80 < a < 200; 50 < b < 400; and 20 < c < 200 µm.

10. Method of implantation of claim 8 or 9 including the further step of implanting the device into a mammal in need thereof.
